# EUROPEAN PATENT APPLICATION

(11) **EP 4 269 393 A1**
(43) Date of publication of application: **01.11.2023**
(21) Application number: 23169661.8
(22) Date of filing: 25.04.2023
(51) Int. Cl.: C07D 215/26, A61P 25/16, A61P 25/28, A61K 31/47, A61K 31/4709

(54) **QUINOLIN-2-YL NITRONES USEFUL FOR THE PREVENTION AND/OR TREATMENT OF NEURODEGENERATIVE DISEASES**

(30) Priority: 25.04.2022 LU 501919
(71) Applicant: Univerza V Ljubljani, 1000 Ljubljana (SI); Consejo Superior de Investigaciones Científicas (CSIC), 28006 Madrid (ES); Universidad Camilo José Cela, 28692 Villanueva de la Cañada (ES)
(72) Inventor: CONTELLES, José Luis Marco, 28029 Madrid (ES); LÓPEZ MUÑOZ, Francisco, 28029 Madrid (ES); KNEZ, Damijan, 1000 Ljubljana (SI); GOBEC, Stanislav, 1000 Ljubljana (SI)
(74) Representative: Zacco GmbH

(57) **Abstract**

The present invention relates to the fields of medicinal chemistry and medicine, and in particular to quinolin-2-yl nitrones with the formula I as pharmaceutically active compounds. These compounds can be used as drugs for the treatment of neurodegenerative diseases by inhibiting BChE or MAO-B, either alone or in combination with other beneficial activities, such as antioxidant properties and metal chelation.

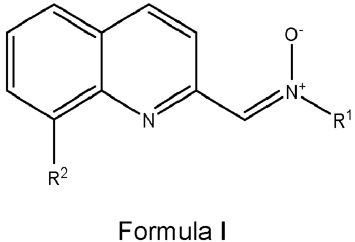

## Description

### Technical Field

The present invention relates to the fields of medicinal chemistry and medicine, and quinolin-2-yl nitrones as pharmacologically active compounds. These compounds can be in the form of a mixture of enantiomers or in the form of pure enantiomers, in the form of pharmaceutically acceptable salts, as hydrates or solvates thereof. The compounds of the present invention are useful for the treatment of neurodegenerative diseases such as Alzheimer's disease, Parkinson's disease and amyotrophic lateral sclerosis.

### Background Art

Alzheimer's disease (AD) is a neurodegenerative disease characterized by progressive deterioration of memory and learning ability due to a variety of pathological changes in the central nervous system (CNS) (Scheltens, P.; Strooper, B. D.; Kivipelto, M.; Holstege, H.; Chételat, G.; Teunissen, C. E.; Cummings, J.; Flier, W. M. van der. The Lancet 2021, 397, 1577-1590). The literature review shows that inhibition of cholinesterases (ChEs), namely acetylcholinesterase (AChE) and butyrylcholinesterase (BChE), monoamine oxidases (MAO-A/B), and β-secretase in addition to modulation of monoaminergic receptors remains the focus of AD-related small molecule drug design (do Carmo Carreiras, M.; Ismaili, L.; Marco-Contelles, J. Bioorg. Med. Chem. Lett. 2020, 30, 126880; Kucwaj-Brysz, K.; Baltrukevich, H.; Czarnota, K.; Handzlik, J. Bioorg. Med. Chem. Lett. 2021, 49, 128275; N. M. Moussa-Pacha, S. M. Abdin, H. A. Omar, H. Alniss, T. H. Al-Tel. Med. Res. Rev. 2020, 40, 339-384; Lalut, J.; Karila, D.; Dallemagne, P.; Rochais, C. Future Med. Chem. 2017, 9, 781-795.) Selective loss of cholinergic neurons leads to a decrease in acetylcholine (ACh) levels in specific brain regions that mediate cognition (Hampel, H.; Mesulam, M.-M.; Cuello, A. C.; Farlow, M. R.; Giacobini, E.; Grossberg, G. T.; Khachaturian, A. S.; Vergallo, A.; Cavedo, E.; Snyder, P. J.; Khachaturian, Z. S. Brain 2018, 141, 1917-1933). Therefore, the inhibition of AChE or BChE increases ACh levels at the synaptic cleft and restores cholinergic neurotransmission (Wang, H.; Zhang, H. ACS Chem. Neurosci. 2019, 10, 852-862). On the other hand, MAOs catalyze the oxidation of amines that act as neurotransmitters, releasing H₂O₂ and consequently reactive oxygen and nitrogen species (Jones, D. N.; Raghanti, M. A. J. Chem. Neuroanat. 2021, 114, 101957). Inhibiting MAO imparts potent neuroprotective effects by decreasing oxidative stress, and restores impaired synaptic plasticity, memory and learning in mouse model of AD via control of tonic GABA levels (Manzoor, S.; Hoda, N. Eur. J. Med. Chem. 2020, 206, 112787; Youdim, M. B. H. J. Neural Transm. 2018, 125, 1719-1733; Jo, S.; Yarishkin, O.; Hwang, Y. J.; Chun, Y. E.; Park, M.; Woo, D. H.; Bae, J. Y.; Kim, T.; Lee, J.; Chun, H.; Park, H. J.; Lee, D. Y.; Hong, J.; Kim, H. Y.; Oh, S.-J.; Park, S. J.; Lee, H.; Yoon, B.-E.; Kim, Y.; Jeong, Y.; Shim, I.; Bae, Y. C.; Cho, J.; Kowall, N. W.; Ryu, H.; Hwang, E.; Kim, D.; Lee, C. J. Nat. Med. 2014, 20, 886-896; Cho, H.-U.; Kim, S.; Sim, J.; Yang, S.; An, H.; Nam, M.-H.; Jang, D.-P.; Lee, C. J. Exp. Mol. Med. 2021, 53, 1148-1158).

Parkinson's disease (PD) is a chronic neurodegenerative disease affecting 1% of people over the age of 60. PD is characterized by the progressive death of dopaminergic neurons in the *substantia nigra,* and the formation of Lewy bodies containing aggregates of α-synuclein (Bloem, B. R.; Okun, M. S.; Klein, C. The Lancet 2021, 397, 2284-2303; Hayes, M. T. Am. J. Med. 2019, 132, 802-807). The deficit in dopaminergic neurotransmission is the basis for the well-known symptoms associated with PD pathology, namely tremor, rigidity, bradykinesia, and postural instability. Approved therapies for PD increase dopamine levels in striatum via selective MAO-B inhibition - for example, rasagiline is prescribed as monotherapy in the early stages of PD and as add-on therapy to levodopa in advanced stages of PD (Armstrong, M. J.; Okun, M. S. JAMA 2020, 323, 548-560). Inhibition of MAO-B is thus a validated approach in PD, while a recently developed reversible MAO-B inhibitor also rescued memory impairment and learning in in APP/PS1 mice model of AD (Park, J.-H.; Ju, Y. H.; Choi, J. W.; Song, H. J.; Jang, B. K.; Woo, J.; Chun, H.; Kim, H. J.; Shin, S. J.; Yarishkin, O.; Jo, S.; Park, M.; Yeon, S. K.; Kim, S.; Kim, J.; Nam, M.-H.; Londhe, A. M.; Kim, J.; Cho, S. J.; Cho, S.; Lee, C.; Hwang, S. Y.; Kim, S. W.; Oh, S.-J.; Cho, J.; Pae, A. N.; Lee, C. J.; Park, K. D. Sci. Adv. 5, eaav0316).

### Summary of Invention

The invention relates to quinolin-2-yl nitrones with the formula I, in the form of pure enantiomers or mixtures of enantiomers, and their pharmaceutically acceptable salts. These compounds can be used as drugs for the treatment of neurodegenerative diseases by inhibiting BChE or MAO-B, either alone or in combination with other beneficial activities, such as antioxidant properties and metal chelation.

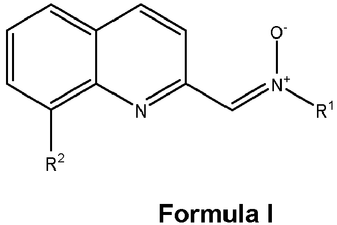

### Brief description of drawings

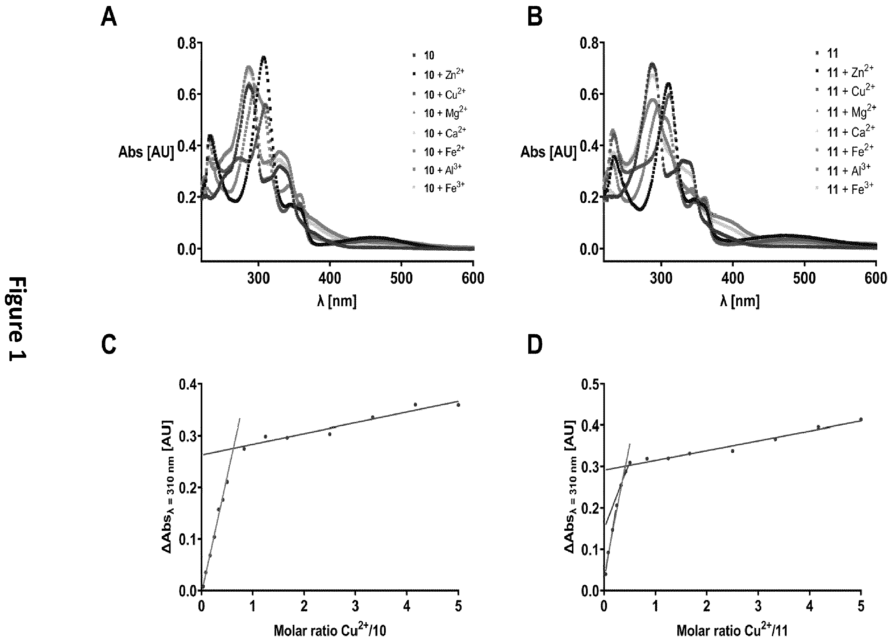

**Figure 1****.** Metal chelating properties of compounds 10 and 11. UV-Vis spectra of QNs **(A)** 10 and **(B)** 11 (30 µM) alone or in the presence of equimolar quantity of metal ions in buffer (20 mM HEPES, 150 mM NaCl, pH = 7.4) at room temperature. UV-vis titration of compounds **(C)** 10 (30 µM) and **(D)** 11 with Cu2+ in buffer.

### Technical Problem

Several drugs have been approved for the treatment of neurodegenerative diseases, but they cause serious side effects and have limited efficacy *in vivo.* Therefore, there is an urgent need for the discovery of new drugs for the treatment of neurodegenerative diseases, especially Alzheimer's disease, Parkinson's disease, and amyotrophic lateral sclerosis.

### Solution to Problem

The above problem is solved by the present invention based on the surprising finding that quinolin-2-yl nitrones with general formula (I) show pharmacological activities benefitial for the treatment of neurodegenerative diseases.

The present invention provides in a first aspect a compound of general formula I 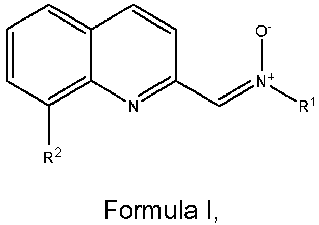 wherein
R¹ represents a -C₁₋₄ alkyl, a substituted or unsubstituted phenyl or a substituted or unsubstituted benzyl, wherein the phenyl or benzyl, if substituted, is substituted by one or more (such as one, two or three) substituents independently selected from -H, -F, -Cl, -Br, -I, -Me, -Et, -Pr, - *i*Pr, -OMe, -OEt, -O*i*Pr, -OH, -NO₂, -NH₂, -CF₃, and -OCF₃;
R² represents -H, -C₁₋₄ alkyl, phenyl, halogen, -OR³ or -NHR³;
R³ represents -H or -C₁₋₄ alkyl, with -C₁₋₄ alkyl being optionally substituted by one or more R⁴ groups;
each R⁴ independently represents halogen, -OH, -OC₁₋₄ alkyl, -NH₂, - NH(C₁₋₄ alkyl) or Cy₁;
Cy₁ represents a 5 to 8-membered ring, saturated, partially unsaturated or aromatic, which contains optionally from 1 to 3 heteroatoms selected among N, O, Se and S; Cy₁ may be attached to rest of the molecule through any C or N atom available, and Cy₁ is optionally substituted by one or more R⁵ groups;
each R⁵ independently represents -C₁₋₄ alkyl optionally substituted by one or more R⁶; and
each R⁶ independently represents -C₂₋₄ alkynyl.

According to some embodiments, R¹ is a -C₁₋₄ alkyl.

According to some embodiments, R¹ is a substituted or unsubstituted phenyl, wherein the phenyl, if substituted, is substituted by one or more (such as one, two or three) substituents independently selected from -H, -F, -Cl, -Br, -I, -Me, -Et, -Pr, -/Pr, -OMe, -OEt, -OiPr, -OH, -NO₂, -NH₂, -CF₃, and -OCF₃;.

According to some embodiments, R¹ is a substituted or unsubstituted benzyl, wherein the benzyl, if substituted, is substituted by one or more (such as one, two or three) substituents independently selected from -H, -F, -Cl, -Br, -I, -Me, -Et, -Pr, -*i*Pr, -OMe, -OEt, -O*i*Pr, -OH, -NO₂, -NH₂, -CF₃, and -OCF₃;.

According to some embodiments, R² is H.

According to some embodiments, R² is -C₁₋₄ alkyl.

According to some embodiments, R² is phenyl.

According to some embodiments, R² is halogen.

According to some embodiments, R² is OR³.

According to some embodiments, R³ in OR³ is H.

According to some embodiments, R³ in OR³ is C₁₋₄ alkyl, optionally substituted by one or more R⁴ groups selected from halogen, -OH, -OC₁₋₄ alkyl, -NH₂, -NH(C₁₋₄ alkyl) or Cy₁.

According to some embodiments, R³ in OR³ is C₁₋₄ alkyl, optionally substituted by Cy₁.

According to some embodiments, Cy₁ represents a 6-membered ring, saturated, partially unsaturated or aromatic, which contains optionally from 1 to 3, such as 1 or 2, heteroatoms selected among N, O, Se and S; Cy₁ may be attached to rest of the molecule through any C or N atom available, and Cy₁ is optionally substituted by one or more R⁵ groups.

According to some embodiments, Cy₁ represents a 6-membered ring, saturated, partially unsaturated or aromatic, which contains optionally from 1 to 3 N atoms, such as 1 or 2 N atoms; Cy₁ may be attached to rest of the molecule through any C or N atom available, and Cy₁ is optionally substituted by one or more R⁵ groups.

According to some embodiments, R² NHR³.

According to some embodiments, R³ in -NHR³ is H.

According to some embodiments, R³ in -NHR³ is C₁₋₄ alkyl, optionally substituted by one or more R⁴ groups selected from halogen, -OH, -OC₁₋₄ alkyl, -NH₂, -NH(C₁₋₄ alkyl) or Cy₁.

According to some embodiments, R³ in -NHR³ is C1-4 alkyl, optionally substituted by Cy₁.

According to some embodiments, Cy₁ represents a 6-membered ring, saturated, partially unsaturated or aromatic, which contains optionally from 1 to 3, such as 1 or 2, heteroatoms selected among N, O, Se and S; Cy₁ may be attached to rest of the molecule through any C or N atom available, and Cy₁ is optionally substituted by one or more R⁵ groups.

According to some embodiments, Cy₁ represents a 6-membered ring, saturated, partially unsaturated or aromatic, which contains optionally from 1 to 3 N atoms, such as 1 or 2 N atoms; Cy₁ may be attached to rest of the molecule through any C or N atom available, and Cy₁ is optionally substituted by one or more R⁵ groups.

Non-limiting examples of compounds of the present invention are:
- compounds of formula I, wherein R¹ is a methyl (CH₃) group, R² is a hydrogen atom, hydroxyl, a methoxy group, -NH-CH₃, or -N-(CH₃)₂;
- compounds of formula I, wherein R¹ is a tert-butyl (t-C₄H₉) group, R² is a hydrogen atom, hydroxyl, a methoxy group, -NH-CH₃, or -N-(CH₃)₂; and
- compounds of formula I, wherein R¹ is a benzyl (CH₂C₆H₅) group, R² is a hydrogen atom, hydroxyl, a methoxy group, -NH-CH₃, or -N-(CH₃)₂.

According to some embodiments, the compound is selected from the group consisting of:
(Z)-N-tert-Butyl-1-(8-(3-(piperidin-1-yl)propoxy)quinolin-2-yl)methanimine oxide;
(Z)-N-Benzyl-1-(8-(3-(piperidin-1-yl)propoxy)quinolin-2-yl)methanimine oxide;
(Z)-N-tert-Butyl-1-(8-(3-(4-(prop-2-yn-1-yl)piperazin-1-yl)propoxy)quinolin-2-yl)methanimine oxide;
(Z)-N-Benzyl-1-(8-(3-(4-(prop-2-yn-1-yl)piperazin-1-yl)propoxy)quinolin-2-yl)methanimine oxide;
(Z)-1-(8-Hydroxyquinolin-2-yl)-N-methylmethanimine oxide;
(Z)-N-tert-butyl-1-(8-hydroxyquinolin-2-yl)methanimine oxide; and
(Z)-N-Benzyl-1-(8-hydroxyquinolin-2-yl)methanimine oxide.

A Particularly preferred compound of the present invention is quinolin-2-yl nitrone (*Z*)-N-benzyl-1-(8-hydroxyquinolin-2-yl)methanimine oxide, which structural formula (formula II) is described below:

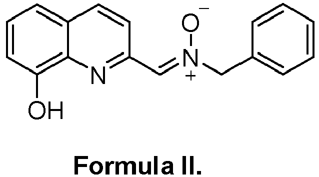

(*Z*)-*N*-Benzyl-1-(8-hydroxyquinolin-2-yl)methanimine oxide showed striking antioxidant capacity against hydroxyl radicals and thus a remarkable activity for neuroprotection of primary cultured neurons after experimental ischemia, as well as a very potent and selective capacity to inhibit human MAO-B and human BChE, as well as strong bio-metal (Zn, Fe, Cu) chelating properties.

Pharmaceutically acceptable pro-drugs, polymorphs, salts and hydrates of any of the above compounds of formula I are included within the present invention.

As used herein, the term "C₁₋₄ alkyl" means a straight or branched chain non-cyclic hydrocarbon having 1, 2, 3 or 4 carbon atoms. The term "C₁₋₄ alkyl" includes methyl, ethyl, propyl, isopropyl, butyl, sec-butyl and tert-butyl. Preferred examples of "C₁₋₄ alkyl" are methyl and tert-butyl.

As used herein, the term "C₂₋₄ alkynyl" means a straight or branched chain non-cyclic hydrocarbon having 2, 3 or 4 carbon atoms and including at least one carbon-carbon triple bond. The term "C₂₋₄ alkynyl" includes ethynyl, 2-propynyl, 3-propynyl, 2-butynyl and 3-butynyl.

It is noted that any of the compounds mentioned as examples throughout the present invention can be used separately or in combination, particularly as adjuvant therapy administered simultaneously, alternatively or successively with respect to a first-line therapy suitable for the treatment of a neurodegenerative disease, such as Alzheimer's disease, Parkinson's disease and amyotrophic lateral sclerosis.

Therefore, in a further aspect the present invention provides a pharmaceutical composition comprising a compound of the present invention, or geometric isomers thereof, and a pharmaceutically acceptable excipient and/or carrier.

In a further aspect the present invention provides a compound or pharmaceutical composition of the present invention for use in medicine.

In a further aspect the present invention provides a compound or pharmaceutical composition of the present invention for use in the treatment of a neurodegenerative disease, such as Alzheimer's disease, Parkinson's disease and amyotrophic lateral sclerosis.

In a further aspect the present invention provides a compound or pharmaceutical composition of the present invention for use as adjuvant therapy.

The adjuvant therapy may be administered simultaneously, alternatively or successively with respect to a first-line therapy suitable for the treatment of a neurodegenerative disease, such as Alzheimer's disease, Parkinson's disease and amyotrophic lateral sclerosis.

The invention is illustrated, though not limited, by the following examples:

### Examples

**General Synthesis Methods.** Reactions were monitored by TLC using precoated silica gel aluminium plates containing a fluorescent indicator (Merck, 5539). Detection was done by UV (254 nm) followed by charring with sulfuric-acetic acid spray, 1% aqueous potassium permanganate solution or 0.5% phosphomolybdic acid in 95% EtOH. Anhydrous Na₂SO₄ was used to dry organic solutions during work-ups and the removal of solvents was carried out under vacuum with a rotary evaporator. Flash column chromatography was performed using silica gel 60 (230-400 mesh, Merck). Melting points were determined on a Kofler block and are uncorrected. IR spectra were obtained on a Perkin-Elmer Spectrum One spectrophotometer. ¹H NMR spectra were recorded with a Varian VXR-200S spectrometer, using tetramethylsilane as internal standard and ¹³C NMR spectra were recorded with a Bruker WP-200-SY. All the assignments for protons and carbons were in agreement with 2D COSY, HSQC, HMBC, and 1D NOESY spectra. Values with (*) can be interchanged. The purity of compounds was checked by elemental analyses, conducted on a Carlo Erba EA 1108 apparatus, and confirmed to be ≥95%.

**General procedure for the synthesis of nitrones.** A solution of the corresponding carbaldehyde (1 mmol), Na₂SO₄ (3 mmol), AcONa (2 mmol) and the appropriate *N*-alkylhydroxylamine hydrochloride (1.5 mmol) in EtOH (5 mL) was heated at 90 °C for 2-3 h under MWI. After that time, the solvent was evaporated and the crude mixture was purified on column chromatography using the indicated mixtures of solvents.

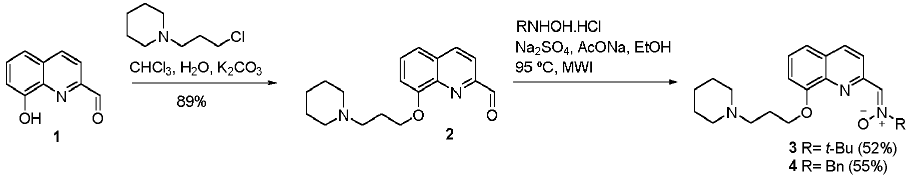

### Synthesis of compounds 3 and 4.

**8-(3-(Piperidin-1-yl)propoxy)quinoline-2-carbaldehyde (2).** A solution of commercial 8-hydroxyquinoline-2-carbaldehyde **(1)** (103.9 mg, 0.6 mmol) in CHCl₃ (3.6 mL)/ water (0,6 mL), K₂CO₃ (249 mg, 1.8 mmol) and commercial 1-(3-chloropropyl)piperidine (178.32 mg, 0.9 mmol) were added. The mixture was vigorously stirred and heated at 80 °C for 1 d. After that time, the solvent was evaporated under reduced pressure and the crude mixture was purified on column chromatography (DCM/MeOH 7%) to yield compound **2** as a yellow solid (158.7 mg, 89%): mp 44-6°C; IR (KBr) ν 2932, 1709, 1462, 1323, 1102 cm⁻¹; ¹H NMR (400 MHz, CDCl₃) δ 10.20 (s, 1H), 8.18 (dd, *J* = 8.5, 0.9 Hz, 1H), 7.97 (d, *J* = 8.4 Hz, 1H), 7.52 (t, *J* = 8.0 Hz, 1H), 7.37 (dd, *J* = 8.3, 1.1 Hz, 1H), 7.12 (dd, *J* = 7.9, 1.2 Hz, 1H), 4.29 (t, *J* = 6.8 Hz, 2H), 2.62 - 2.51 (m, 2H), 2.50 - 2.30 (m, 4H), 2.25 - 2.12 (m, 2H), 1.55 (p, *J* = 5.6 Hz, 4H), 1.39 (q, *J* = 5.7, 4.3 Hz, 2H); ¹³C NMR (101 MHz, CDCl₃) δ 193.9, 155.5, 151.4, 140.1, 137.2, 131.4, 129.8, 119.5, 117.7, 109.9, 67.9, 55.78, 54.6 (2C), 26.33, 25.8 (2C), 24.3. HRMS (ESI_ACN) Calcd. for C₁₈H₂₂N₂O₂: 298.1682. Found: 298.1690.

**(Z)-*N*-*tert*-Butyl-1-(8-(3-(piperidin-1-yl)propoxy)quinolin-2-yl)methanimine oxide (3).** Following the general method for the synthesis of nitrones, a solution of carbaldehyde **2** (79.35 mg, 0.27 mmol), Na₂SO₄ (76.68 mg, 0.54 mmol), AcONa (26.57 mg, 0.324 mmol) and *N-tert-*butylhydroxylamine hydrochloride (40.69 mg, 0.324 mmol) in EtOH (5 mL) was heated at 95 °C for 3 h under MWI. Then, the solvent was evaporated and the crude mixture was purified on column chromatography (DCM/MeOH 7%) to yield compound **3** as a yellow solid (52.3 mg, 52%): mp 135-7 °C; IR (KBr) v 3493, 2942, 1615, 1261, 1096 cm⁻¹; ¹H NMR (400 MHz, CDCl₃) δ 9.27 (d, *J* = 8.8 Hz, 1H), 8.36 - 8.13 (m, 1H), 7.53 - 7.41 (m, 2H), 7.16 - 7.04 (m, 1H), 4.37 (t, *J* = 5.7 Hz, 2H), 3.61 (d, *J* = 11.9 Hz, 2H), 3.43 - 3.28 (m, 2H), 2.68 (dd, *J* = 15.8, 6.7 Hz, 4H), 2.32 (d, *J* = 13.8 Hz, 2H), 2.00 - 1.79 (m, 4H), 1.67 (s, 9H); ¹³C NMR (101 MHz, CDCl₃) δ 137.5, 132.1, 129.6, 127.6, 127.5, 121.9, 120.4, 119.1, 110.2, 110.0, 72.5. 66.7, 55.4, 53.5 (2C), 28.4 (3C), 23.9, 22.7 (2C), 22.2. HRMS (ESI_ACN) Calcd. for C₂₂H₃₁N₃O₂: 369.24163. Found: 369.24136.

**(Z)-*N*-Benzyl-1-(8-(3-(piperidin-1-yl)propoxy)quinolin-2-yl)methanimine oxide (4).** Following the general method for the synthesis of nitrones, a solution of carbaldehyde **2** (79.35 mg, 0.27 mmol), Na₂SO₄ (76.68 mg, 0.54 mmol), AcONa (26.57 mg, 0.324 mmol) and *N-*benzylhydroxylamine hydrochloride (51.71 mg, 0.324 mmol) in EtOH (5 mL) was heated at 90 °C for 2 h under MWI. After that time, the solvent was evaporated and the crude mixture was purified on column chromatography (DCM/MeOH 7%) to yield compound **4** as a mixture of *Z* and *E* isomers in a 3.5:1 ratio, that we were unable to separate, as a yellow solid (59.7 mg, 55%): mp 108-10 °C; IR (KBr) v 3420, 2935, 1600, 1455, 1105 cm⁻¹; ¹H NMR (400 MHz, CDCl₃) **[(minor isomer *E*)** 8.17 (d, *J* = 8.8 Hz, 1H, H4), 7.79 (s, 1H, CH=N), 7.74 (dd, *J* = 8.4, 1.1 Hz, 1H, H3), 7.55 (dd, *J*= 7.0, 2.5 Hz, 1H, H5), 7.09 (br d, *J*= 1.5 Hz, 1H, H7), 5.43 (s, 1H, C*H*₂C₆H₅), **(major isomer *Z*)** 9.15 (d, *J* = 8.8 Hz, 1H, H4), 8.17 (d, *J* = 8.8 Hz, 1H, H3), 8.08 (s, 1H, CH=N), 7.07 (br d, *J*= 7.5 Hz, 1H, H7), 5.15 (s, 1H, C*H*₂C₆H₅)], 8.28 - 8.26 (m, 1H, C₆H₅), 7.54 (br d, *J*= Hz, 1H, H5), 7.44 - 7.38 (m, 6H, H6, C₆H₅), 7.45 (t, *J*= Hz, 1H, H6), 7.52 - 7.37 (m, 4H, C₆H₅), 4.31 (t, *J*= Hz., 2H, OCH₂), 3.15 - 2.65 (m, 6H), 2.43-2.41 (m, 2H), 1.98-1.70 (m, 4 H), 1.65-1.51 (m, 2H). HRMS (ESI_ACN) Calcd. for C₂₅H₂₉N₃O₂: 403.22598. Found: 403.22528.

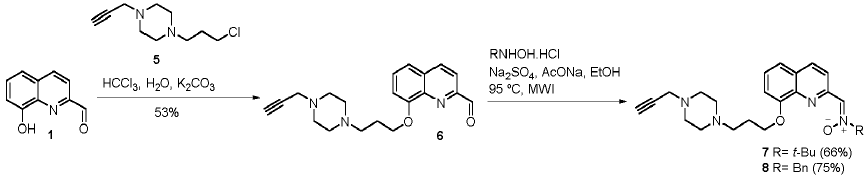

### Synthesis of QNs 7 and 8.

**1-(3-Chloropropyl)-4-(prop-2-yn-1-yl)piperazine (5).** To solution of commercial 1-(3-chloropropyl)piperazine dihydrochloride (702 mg, 3 mmol, 1 equiv), TEA (0,84 mL, 6 mmol, 2 equiv) in dry CH₂Cl₂ (5 mL), cooled at 0 °C, propargyl bromide (0,81 mL, 9 mmol, 3 equiv) was added over 30 min under Ar. The mixture was stirred at rt for 24 h and then treated with a saturated sodium bicarbonate solution (10 mL). The organic layer was separated and washed with saturated brine, and dried over anhydrous sodium sulfate. The solvent was removed under reduced pressure and the residue purified by column chromatography through silica gel (CH₂Cl₂/MeOH 1%-2%), to yield **5** (312 mg, 52%) as a colorless oil: ¹H NMR (400 MHz, CDCl₃) δ 3.53 (t, *J* = 6.6 Hz, 2H), 3.23 (d, *J* = 2.5 Hz, 2H), 2.54 - 2.39 (m, 10H), 2.18 (t, *J* = 2.5 Hz, 1H), 1.88 (p, *J* = 6.7 Hz, 2H); ¹³C NMR (101 MHz, CDCl₃) δ 78.8, 73.2, 55.4, 53.1 (2C), 51.9 (2C), 46.8, 43.2, 29.9.

**8-(3-(4-(Prop-2-yn-1-yl)piperazin-1-yl)propoxy)quinoline-2-carbaldehyde (6).** A solution of commercial 8-hydroxyquinoline-2-carbaldehyde **(1)** (91.69 mg, 0.53 mmol) in CHCl₃ (5 mL) was added K₂CO₃ (219.42 mg, 1.59 mmol) and 1-(3-chloropropyl)-4-(prop-2-yn-1-yl)piperazine **(5)** (138 mg, 0.69 mmol); then, water (1 mL) was added. The mixture was vigorously stirred and heated at 80 °C for 2 d. After that time, the solvent was evaporated under reduced pressure and the crude mixture was purified on column chromatography (DCM/MeOH 4%) to yield compound **6** as a yellow solid (95.5 mg, 53%): mp 99-101°C; IR (KBr) v 3128, 2829, 1715, 1462, 1320, 1094 cm⁻¹; ¹H NMR (400 MHz, CDCl₃) δ 10.20 (s, 1H), 8.20 (d, *J* = 8.4 Hz, 1H), 7.98 (d, *J* = 8.5 Hz, 1H), 7.53 (t, *J* = 8.5 Hz, 1H), 7.39 (d, *J* = 8.5 Hz, 1H), 7.13 (d, *J* = 8.4 Hz, 1H), 4.31 (t, *J* = 6.5 Hz, 2H), 3.24 (s, 2H), 2.76 - 2.36 (m, 10H), 2.26 - 2.13 (m, 3H); ¹³C NMR (101 MHz, CDCl₃) δ 193.8, 155.5, 151.4, 140.1, 137.2, 131.4, 129.8, 119.6, 117.8, 110.0, 78.8, 73.2, 67.7, 55.0, 53.0 (2C), 51.8 (2C), 46.8, 26.3. HRMS (ESI_ACN) Calcd. for C₂₀H₂₃N₃O₂: 337.1790. Found: 337.1790.

**(*Z*)-*N*-*tert*-Butyl-1-(8-(3-(4-(prop-2-yn-1-yl)piperazin-1-yl)propoxy)quinolin-2-yl)methanimine oxide (7).** Following the general method for the synthesis of nitrones, the reaction of carbaldehyde **6** (69 mg, 0.20 mmol) with Na₂SO₄ (57 mg, 0.4 mmol), AcONa (26 mg, 0.32 mmol) and N-tert-butylhydroxylamine hydrochloride (38 mg, 0.3 mmol) in EtOH (5 mL) after 5 min, and column chromatography (DCM/MeOH 3%) yielded compound **7** as a yellow gum (55.7 mg, 66%): IR (KBr) v 3428, 2819, 1451, 13206, 1155, 1104 cm⁻¹; ¹H NMR (400 Hz, CDCl₃) δ 9.26 (d, *J* = 8.8 Hz, 1H), 8.19 (d, *J* = 8.8 Hz, 1H), 8.13 (s, 1H), 7.44-7.38 (m, 2H,), 7.08-7.06 (m, 1H), 4.29 (t, *J* = 6.9 Hz, 2H), 3.30-3.28 (m, 2H), 2.63 (s, 10H), 2.32 - 2.17 (m, 3H), 1.65 (s, 9H); ¹³C NMR (126 MHz, CDCl₃) δ 154.6, 149.8, 140.2, 136.6, 132.6, 129.5, 127.3, 121.7, 119.5, 109.0, 78.7, 73.4, 72.0, 67.7, 55.0, 53.0 (2C), 51.8 (2C), 46.8, 28.3 (3C), 26.3. HRMS (ESI_ACN) Calcd for C₂₄H₃₂N₄O₂: 408,2525. Found: 408,2525.

**(Z)-*N*-Benzyl-1-(8-(3-(4-(prop-2-yn-1-yl)piperazin-1-yl)propoxy)quinolin-2-yl)methanimine oxide (8).** Following the general method for the synthesis of nitrones, a solution of carbaldehyde **6** (95.5 mg, 0.28 mmol), treated with Na₂SO₄ (79.52 mg, 0.56 mmol), NaHCOs (35.28 mg, 0.42 mmol) and *N*-benzylhydroxylamine hydrochloride (67.03 mg, 0.42 mmol), in THF (5 mL), reacted instantly. After that time, the solvent was evaporated and the crude mixture was purified on column chromatography (DCM/MeOH 3%) to yield compound **8** as a mixture of *E* and *Z* isomers in a 1.5:1 ratio, that we were unable to separate, as a yellow gum (92.5 mg, 75%): IR (KBr) v 3429, 2817, 1457, 1320, 1151, 1104 cm⁻¹; ¹H NMR (500 MHz, CDCl₃) δ **[(major isomer *E*)** 8.17 (d, *J* = 8.5 Hz, 1H, H4), 7.81 (d, *J* = 8.5 Hz, 1H, H3), 7.75 (s, 1H, CH=N), 7.55 (dd, *J*= 7.8, 1.7 Hz, 1H, H5), 7.12 (dd, *J*= 7.7, 1.2 Hz, 1H, H7), 5.44 (s, 1H, CH₂C₆H₅), 3.30 (d, *J* = 2.4 Hz, 2H, *CH*₂C≡CH), **(minor isomer *Z*)** 9.18 (d, *J* = 8.7 Hz, 1H, H4), 8.19 (d, *J* = 8.7 Hz, 1H, H3), 8.06 (s, 1H, CH=N), 7.10 (dd, *J*= 7.7, 1.3 Hz, 1H, H7), 5.14 (s, 1H, C*H*₂C₆H₅), 3.31 (d, *J* = 2.4 Hz, 2H, C*H*₂C≡CH)], 8.26 - 8.24 (m, 1H, C₆H₅), 7.45 (t, *J*= Hz, 1H, H6), 7.52 - 7.37 [m, 4H, H5 (minor)], C₆H₅), 4.31 (t, *J*= Hz, 2H, OCH₂), 2.75 - 2.50 (m, 10H), 2.25-2.24 (m, 1H, CH₂C≡C*H*), 2.24-2.16 (h, *J*= Hz, 2H, NCH₂C*H*₂CH₂O). HRMS (ESI_ACN) Calcd for C₂₇H₃₀ClN₄O₂: 442.2369. Found: 442.2369.

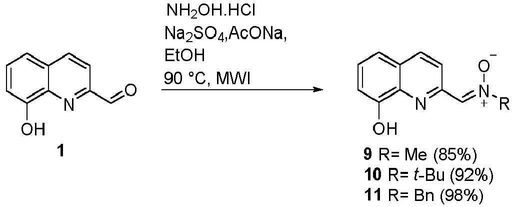

### Synthesis of QNs 9-11.

**(*Z*)-1-(8-Hydroxyquinolin-2-yl)-*N*-methylmethanimine oxide (9).** Following the general method for the synthesis of nitrones, the reaction of 8-hydroxyquinoline-2-carbaldehyde **(1)** (173 mg, 1 mmol, 1 equiv) with Na₂SO₄ (426 mg, 3 mmol, 3 equiv), AcONa (160 mg, 2 mmol, 1.6 equiv) and *N*-methylhydroxylamine hydrochloride (239 mg, 1.5 mmol, 1.5 equiv) in EtOH (7 mL) at 90 °C for 10 min, after column chromatography (hexane/AcOEt 9/1) yielded compound **9** as a pale yellow solid (171 mg, 85%): 149-151 °C; ¹H NMR (300 MHz, CDCl₃) δ 9.16 (d, *J* = 8.8 Hz, 1H), 8.25 (d, *J* = 8.8 Hz, 1H), 8.03 (br s, 1H), 7.86 (s, 1H), 7.51-7.46 (m, 1H), 7.36 (dd, *J* = 7.6, 1.3 Hz, 1H), 7.18 (dd, *J* = 7.6, 1.3 Hz, 1H), 4.01 (s, 3H); ¹³C NMR (100 MHz, CDCl₃) δ 152.1, 147.7, 138.1, 136.8, 136.4, 128.7, 128.6, 121.4, 117.8, 110.1, 55.1. HRMS (ESI_ACN) Calcd. for C₁₁H₁₀N₂O₂: 202,0742. Found 202,0742.

**(*Z*)-*N*-*tert*-butyl-1-(8-hydroxyquinolin-2-yl)methanimine oxide (10).** Following the general method for the synthesis of nitrones, the reaction of 8-hydroxyquinoline-2-carbaldehyde **(1)** (173 mg, 1 mmol, 1 equiv) with Na₂SO₄ (426 mg, 3 mmol, 3 equiv), AcONa (160 mg, 2 mmol, 1.6 equiv) and *N*- *tert*-butylhydroxylamine hydrochloride (188 mg, 1.5 mmol, 1.5 equiv) in EtOH (7 mL) at 90 °C for 5 min, after column chromatography (hexane/AcOEt 9/1), yielded compound **10** as a pale yellow solid (159 mg, 92%): 103-4 °C; ¹H NMR (400 MHz, CDCl₃) δ 9.16 (d, *J* = 8.8 Hz, 1H), 8.16 (d, *J* = 8.8 Hz, 1H), 8.04 (br s , 1H), 7.97 (s, 1H), 7.41-7.39 (m, 1H), 7.27 (dd, *J* = 7.6, 1.2 Hz, 1H), 7.09 (dd, *J* = 7.6, 1.2 Hz, 1H), 1.62 (s, 9H); ¹³C NMR (100 MHz, CDCl₃) δ 152.0, 148.5, 138.1, 136.7, 131.5, 128.5, 128.4, 121.7, 117.8, 110.0, 72.1, 28.4 (3C). HRMS (ESI_ACN) Calcd. for C₁₄H₁₆N₂O₂: 244,1212. Found 244,1212.

**(*Z*)-*N*-Benzyl-1-(8-hydroxyquinolin-2-yl)methanimine oxide (11).** Following the general method for the synthesis of nitrones, the reaction of 8-hydroxyquinoline-2-carbaldehyde **(1)** (173 mg, 1 mmol, 1 equiv), Na₂SO₄ (426 mg, 3 mmol, 3 equiv.), AcONa (160 mg, 2 mmol, 1.6 equiv.) and *N-*benzylhydroxylamine hydrochloride (239 mg, 1.5 mmol, 1.5 equiv.) in EtOH (7 mL) was heated at 90°C during 10 min under mw irradiation. After that time, the solvent was evaporated and the crude mixture was purified on column chromatography (Hexanes/AcOEt 9/1) to yield compound **11** as a pale yellow solid (272 mg, 98%): 110-1 °C; ¹H NMR (400 MHz, CDCl₃) δ 9.15 (d, *J* = 8.8 Hz, 1H), 8.22 (d, *J* = 8.8 Hz, 1H), 8.00 (br s, 1H), 7.88 (s, 1H), 7.55-7.51(m, 2H), 7.44-7.37 (m, 4H), 7.26 (dd, *J* = 7.6, 1.2 Hz, 1H), 7.08 (dd, *J* = 7.6, 1.2 Hz, 1H), 5.09 (s, 2H); ¹³C NMR (100 MHz, CDCl₃) δ 152.1, 147.7, 138.1, 136.8, 135.3, 132.7, 129.5 (2C), 129.3, 129.2 (2C), 128.7, 128.6, 121.6, 117.8, 110.1, 72.1. HRMS (ESI_ACN) Calcd. for C₁₇H₁₄N₂O₂: 278,1055. Found 278,1055.

### Pharmacological evaluation

**Inhibition of Cholinesterases.** The inhibitory potencies of compounds against the ChEs were determined by the method of Ellman (Ellman, G. L.; Courtney, K. D.; Andres, V.; Featherstone, R. M. Biochem. Pharmacol. 1961, 7, 88-95). Briefly, compounds were incubated with Ellman's reagent (final concentration, 370 µM) and the ChEs (final concentration, approx. 1 nM or 100 pM hBChE or hAChE, respectively) in 0.1 M sodium phosphate (pH 8.0) for 5 min at 20 °C on 96-well microplates (Brand microplate, pureGrade, F-bottom). Reactions were started adding the substrate (final concentration, 500 µM butyrylthiocholine iodide [BTCI] or acetylthiocholine iodide [ATCI] for hBChE and hAChE, respectively). The final content of DMSO was always 1% (v/v). The increase in absorbance (λ = 412 nm) was monitored for 2 min using a microplate reader (Synergy HT, BioTek Instruments, VT, USA). The initial velocities in the presence (vi) and absence (vo) of the compounds were calculated, and the inhibitory potencies were expressed as residual activities (RA = vi / vo). The IC₅₀ values were determined by plotting RA against the applied inhibitor concentrations, with the experimental data fitted to a four-parameter logistic function (GraphPad Prism 9.3, GraphPad Software, San Diego, CA, USA). Donepezil was used as a positive control (Table 1).

**Inhibition of Monoamine Oxidases.** Recombinant microsomal hMAOs expressed in BTI-TN-5B1-4 cells, HRP (type II, lyophilized powder) and p-tyramine hydrochloride were purchased from Sigma Aldrich (Sigma Aldrich, MO, USA). 10-Acetyl-3,7-dihydroxyphenoxazine (Amplex Red) was synthesized as described previously (von der Eltz, H.; DE; Guder, H.-J.; DE; Muhlegger, K.; DE. United States Patent: 5035998 - Hydrolase Substrates. 5035998, July 30, 1991). Briefly, 100 µL of 50 mM sodium phosphate (pH 7.4, 0.05% [v/v] Triton X-114) containing the test compounds, and hMAO-A or hMAO-B were incubated at 37 °C for 15 min in 96-well microplates (Nunc Microwell microplates, Thermo Fisher). After preincubation, the reaction was started by adding Amplex Red (final concentration, 200 µM), HRP (2 U/mL), and p-tyramine (1 mM). The increase in fluorescence intensity (λex = 530 nm, λem = 590 nm) was monitored at 37 °C for 30 min using a microplate reader (Synergy HT; BioTek Instruments, Inc., VT, USA). DMSO was used for control experiments (1%, v/v). To determine the blank value (b), sodium phosphate buffer replaced the enzyme solution. Initial velocities were calculated from the trends obtained, with each measurement performed in duplicate. The inhibitory potencies are expressed as the RAs according to equation: RA = (vi - b) / (vo - b), where vi is the velocity in the presence of the test compounds, and v0 is the control velocity in the presence of DMSO. IC₅₀ values were determined by plotting the residual MAO activities against the applied inhibitor concentrations, with the experimental data fitted to a Hill four-parameter equation (GraphPad Prism 9.3, GraphPad Software, San Diego, CA, USA). For the reversibility assay, hMAO-B was incubated at 100-fold final concentration with the inhibitors at 10-fold IC50 concentration at 37 °C (volume, 50 µL). After 15 min, the mixture was diluted 100-fold into the reaction buffer containing Amplex Red, HRP, and p-tyramine hydrochloride. The final concentrations of all reagents and of hMAO-B were the same as described above. Control experiments were performed in the same manner, replacing the inhibitor solution with DMSO (Table 1).

**Table 1. In vitro MAO and ChE inhibitory potencies of compounds and positive controls.**

| **Compound Structure** | | **IC₅₀ ± SEM [µM]** ^{a} | | | |
|---|---|---|---|---|---|
| | | **hMAO-A** | **hMAO-B** | **hAChE** | **hBChE** |
| **3** | 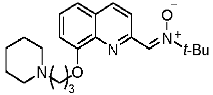 | n.a.*^{b}* | n.a.*^{b}* | n.a.^{*b*,*c*} | **0.0254 ± 0.0021** |
| **4** | 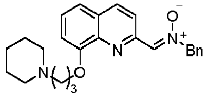 | n.a.*^{b}* | n.a.*^{b}* | n.a.^{*b*,*c*} | **0.0769 ± 0.0063** |
| **7** | 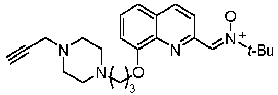 | n.a.*^{b}* | n.a.*^{b}* | n.a.*^{b}* | **0.0073 ± 0.0011** |
| **8** | 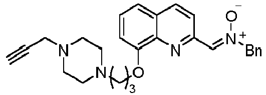 | n.a.^{*b*,*c*} | n.a.*^{b}* | n.a.*^{b}* | **0.0310 ± 0.0064** |
| **9** | 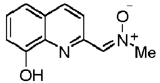 | n.a.^{*b*,*c*} | **10.1 ± 2.5** | n.a.*^{b}* | n.a.*^{b}* |
| **10** | 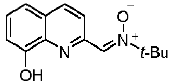 | n.a.*^{b}* | **16.4 ± 1.0** | n.a.*^{b}* | **0.0051 ± 0.0006** |
| **11** | 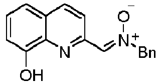 | n.a.*^{b}* | **4.46 ± 0.18** | **0.0290± 0.0030** | **0.0011 ± 0.00031 *^{d}*** |
| **Donepezil** | 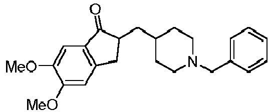 | n.d.*^{e}* | n.d.*^{e}* | **0.0220± 0.0024** | **4.15 ± 0.56** |
| **Safinamide** | 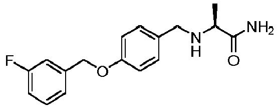 | n.a.*^{b}* | **0.029± 0.002** | n.d.*^{e}* | n.d.*^{e}* |

| | | | | | |
|---|---|---|---|---|---|
| *^{a}* SEM, standard error of the mean, IC₅₀ values are average of three independent experiments, each performed in triplicate; *^{b}* n.a., not active (residual activity - RA at 100 µM ≥50%). *^{c}* nonspecific inhibition at the screening concentration (100 µM) due to solubility issues, inhibition disappears upon dilution (10 µM). *^{d}* IC₅₀ value do not reflect the true affinity of the compound due to the IC₅₀ value approaching the concentration of the hBChE (approx. 1 nM) in the *in vitro* assays. | | | | | |

**DPPH radical-scavenging potency of compounds 10 and 11.** Free-radical scavenging potency was evaluated using the DPPH assay. DPPH (2,2-diphenyl-1-picrylhydrazyl radical) was dissolved in MeOH (150 µL, 140 µM) and added to 150 µL methanol solution of the test sample (screening at 100 µM, serial dilution of compounds for EC₅₀ determination) or methanol (negative control) on 96-well microtiter plates (Brand microplate, pureGrade, F-bottom). The microtiter plate was incubated at room temperature in the dark for 90 min. The absorbance at 517 nm was then determined with a microplate reader (Synergy HT; BioTek Instruments, Inc., VT, USA). The experiments were performed in triplicate, with subtraction of the blank value (compound without DPPH). The percentages of DPPH free radicals were calculated as DPPH free radical (%) = [(A0 - A1) / A0] × 100, where A0 is the absorbance of the negative control, and A1 is the absorbance of the test sample. The free-radical scavenging potency is expressed as the concentration that scavenged 50% of the DPPH free radicals (EC₅₀) ± SEM. Compounds **10** and **11** displayed the free-radical scavenging potency with EC₅₀ values of 119.2±1.2 µM and 126.0±0.1 µM, respectively. Resveratrol and Trolox were used as the positive controls under the same assay conditions.

**Metal-chelating properties of 10 and 11.** The chelation properties were determined in HEPES buffer (20 mM, 150 mM NaCl, pH 7.4) using a 96-well microplate reader (Synergy HT; BioTek Instruments, Inc., VT, USA). To determine the chelation of the metal ions by the compounds, 30 µM compound solution was treated with equimolar concentrations of CuCl₂, ZnCl₂, CoCl₂, MgCl₂, CaCl₂, FeCl₂, FeCl₃, and AlCl₃. To prevent oxidation of Fe²⁺, the solution of FeCl₂ was prepared in the presence of 1 mM ascorbic acid. The absorption spectra were recorded after 30 min incubation at room temperature. The chelation was detected by the change in the absorption spectra, which was specific for each metal ion. The Cu²⁺ binding stoichiometry was resolved by titration of a 30 µM buffered solution of the compounds with additions of CuCl₂ buffered stock solution. The absorption of the compounds in the absence and presence of increasing Cu²⁺ concentrations (0-150 µM) were recorded at the most responsive wavelength after 30 min incubation at room temperature. The absorbance differences in the absence and presence of Cu²⁺ were plotted against the Cu²⁺/compound molar ratio. The curves were approximated using data points at the lowest and highest Cu²⁺/compound ratios, and the intercepts were calculated (Yoe-Jones method) (Bosque-Sendra, J. M.; Almansa-López, E.; García-Campaña, M.; Cuadros-Rodríguez, L. Anal. Sci. 2003, 19, 1431-1439).

The absorption spectra of **10** (λabs, max = 286 nm) incubated with equimolar quantity of Zn²⁺, Cu²⁺, and Al³⁺ showed characteristic bathochromic shift with new absorption maxima at 309, 310 and 296 nm, respectively (Figure 1A), whereas addition of Fe²⁺ and Fe³⁺ resulted in hyperchromic shift, i.e., the increase in absorbance at 286 nm, and appearance of secondary maxima at ~230 nm. On the other hand, no major changes in absorption spectra were observed for Ca²⁺ and Mg²⁺ ions. Similar was observed for nitrone 19 (λabs, max = 287 nm): i) appearance of maxima at 310, 311 and 296 nm for Zn²⁺, Cu²⁺, and Al³⁺, respectively; ii) hypochromic change in absorbance spectra for 19 in the presence of Fe²⁺ and Fe³⁺, and iii) no major alterations in spectra upon incubation with Ca²⁺ and Mg²⁺ (Figure 1B). This indicated that both QNs generated corresponding complexes with Zn²⁺, Cu²⁺, Al³⁺, Fe²⁺ and Fe³⁺, whereas they did not chelate Ca²⁺ and Mg₂₊. Copper ion is of particular interest in AD,59,60 thus the stoichiometry of the Cu2+ complexes with compounds **10** and **11** was determined by Yoe-Jones method,61 which showed an interception point at 0.5 indicating **10**-Cu²⁺ and **11**-Cu²⁺ complexes with a 1:2 Cu²⁺/compound molar ratio (Figure 1).

## Claims

1. Compound of formula I, 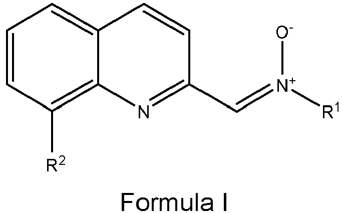 wherein
R¹ represents a -C₁₋₄ alkyl, a substituted or unsubstituted phenyl or a substituted or unsubstituted benzyl, wherein the phenyl or benzyl, if substituted, is substituted by one or more (such as one, two or three) substituents independently selected from -H, -F, -Cl, -Br, -I, -Me, -Et, -Pr, - *i*Pr, -OMe, -OEt, -OiPr, -OH, -NO₂, -NH₂, -CF₃, and -OCF₃;
R² represents -H, C₁₋₄ alkyl, phenyl, halogen, -OR³ or -NHR³;
R³ represents -H or -C₁₋₄ alkyl, and -C₁₋₄ alkyl is optionally substituted by one or more R⁴ groups;
each R⁴ independently represents halogen, -OH, -OC₁₋₄ alkyl, -NH₂, - NH(C₁₋₄ alkyl) or Cy₁; and
Cy₁ represents a 5 to 8 member ring, saturated, partially insaturated or aromatic, which contains optionally from 1 to 3 heteroatoms selected among N, O, Se and S; Cy₁ may be attached to rest of the molecule through any C or N atom available, and Cy₁ is optionally substituted by one or more R⁵ groups;
each R⁵ independently represent -C₁₋₄ alkyl optionally substituted by one or more R⁶; and
each R⁶ independently represents -C₂₋₄ alkynyl,
or a pharmaceutically acceptable salt or hydrate thereof.

2. Compound according to claim 1, wherein R¹ is -C₁₋₄ alkyl.

3. Compound according to claim 1 or 2, wherein R¹ is a methyl (CH₃) group or *tert*-butyl (*t*-C₄H₉) group.

4. Compound according to claim 1, wherein R¹ is a benzyl (CH₂C₆H₅) group.

5. Compound according to any one of claims 1 to 4, wherein R² is a hydrogen atom, hydroxyl, a methoxy group, -NH-CH₃, or -N-(CH₃)₂.

6. Compound according to claim 1, wherein R¹ is a methyl (CH₃) group, and R² is a hydrogen atom, hydroxyl, a methoxy group, -NH-CH₃, or -N-(CH₃)₂.

7. Compound according to claim 1, wherein R¹ is a *tert*-butyl (*t*-C₄H₉) group, and R² is a hydrogen, hydroxyl, a methoxy group, -NH-CH₃, or -N-(CH₃)₂.

8. Compound according to claim 1, wherein R¹ is a benzyl (CH₂C₆H₅) group, and R² is a hydrogen, hydroxyl, a methoxy group, -NH-CH₃, or -N-(CH₃)₂.

9. Compound according to claim 1, selected from the group consisting of:
(Z)-N-tert-Butyl-1-(8-(3-(piperidin-1-yl)propoxy)quinolin-2-yl)methanimine oxide;
(Z)-N-Benzyl-1-(8-(3-(piperidin-1-yl)propoxy)quinolin-2-yl)methanimine oxide;
(Z)-N-tert-Butyl-1-(8-(3-(4-(prop-2-yn-1-yl)piperazin-1-yl)propoxy)quinolin-2-yl)methanimine oxide;
(Z)-N-Benzyl-1-(8-(3-(4-(prop-2-yn-1-yl)piperazin-1-yl)propoxy)quinolin-2-yl)methanimine oxide;
(Z)-1-(8-Hydroxyquinolin-2-yl)-N-methylmethanimine oxide;
(Z)-N-tert-butyl-1-(8-hydroxyquinolin-2-yl)methanimine oxide; and
(Z)-N-Benzyl-1-(8-hydroxyquinolin-2-yl)methanimine oxide.

10. Compound according to claim 1, wherein said compound is (*Z*)-N-benzyl-1-(8-hydroxyquinolin-2-yl)methanimine oxide.

11. The compound according to any one of claims 1 to 10 for use in medicine.

12. The compound according to any one of claims 1 to 10 for use in the prevention and/or treatment of a neurodegenerative disease.

13. A pharmaceutical composition comprising a compound according to any of claims 1 to 10, and a pharmaceutically suitable excipient and/or carrier.

14. The pharmaceutical composition according to claim 13 for use in medicine.

15. The pharmaceutical composition according to claim 13 for use in the prevention and/or treatment of a neurodegenerative disease.

16. The compound for use according to claim 12 or the pharmaceutical composition for use according to claim 15, wherein for the neurodegenerative disease is Alzheimer's disease, Parkinson's disease amyotrophic lateral sclerosis.

17. The compound according to any one of claims 1 to 10 or the pharmaceutical composition according to claim 13 for use as adjuvant therapy for a neurodegenerative disease.
